Europäisches Patentamt

European Patent Office

Office européen des brevets

(11) Publication number: **0 032 828**
A2

## (12) EUROPEAN PATENT APPLICATION

(21) Application number: **81300204.5**

(22) Date of filing: **16.01.81**

(51) Int. Cl.³: **A 61 F 1/03** ·

(30) Priority: **17.01.80 GB 8001597**

(43) Date of publication of application: **29.07.81**
**Bulletin 81/30**

(84) Designated Contracting States: **AT BE CH DE FR GB IT
LI LU NL SE**

(71) Applicant: **HOWMEDICA INTERNATIONAL INC.,
Shannon Industrial Estate, Shannon Co. Clare (IE)**

(72) Inventor: **Lindstrand, Anders, Valthornsvagen 13,
S-223 68 Lund (SE)**
Inventor: **Jonsson, Gunnar, c/o Gudlaugur E. Jonsson
Heidargerdi 116, Reykjavik (IS)**
Inventor: **Stenstrom, Anders, Korsakersvagen 89,
S-222 50 Lund (SE)**

(74) Representative: **Bridge-Butler, Alan James et al, G.F.
REDFERN & CO. High Holborn House 52/54 High
Holborn, London WC1V 6RL (GB)**

(54) **Tibial prosthesis component and total prosthesis devices employing such components.**

(57) A tibial prosthesis component to replace a tibial condylar surface of a natural knee joint, said component comprising a main portion adapted for connection to the tibia and a bearing portion made from a plastics material the upper surface of which is adapted to co-operate with a femoral condylar surface of the natural joint or a prosthetic component replacing it, said bearing portion being deformable to provide a releasable snap fit connection to said main portion.

Tibial Prosthesis Component and Total Prosthesis Devices Employing Such Components.

This invention relates to a tibial prosthesis component to replace a tibial condylar surface of a natural knee joint and to total prosthesis devices employing such a tibial component or components.

It is known to provide endo-prosthetic devices for replacing the condylar surfaces of a natural knee joint but such devices, if they include a removable bearing surface on the tibial component are complicated and are usually made from a number of separate parts which require manipulation by the surgeon.

The present invention is intended to provide a tibial prosthesis component which consists of the minimum number of separate parts and is easy for the surgeon to assemble.

The reason for the tibial component being in separate pieces is because it is advantageous to provide a bearing surface of a plastics material for co-operation with a metal femoral condylar surface on a femoral component of the joint. Unfortunately such plastics materials are not sufficiently strong in themselves to carry the bearing loads and it is therefore necessary to support them on a metal, or other material support which can be connected to the tibia. A further advantage of using a separate member having the bearing surface is that when it becomes worn it can be replaced.

According to the present invention a tibial prosthesis component to replace a tibial condylar surface of a natural knee joint comprises a main portion adapted for connection to the tibia and a bearing portion made from a plastics material the upper surface of which is adapted to co-operate with a femoral condylar surface of the natural joint or a prosthetic component replacing it, said bearing portion being deformable to provide a releasable snap fit connection to said main portion.

The term "snap fit" is used herein to mean that one portion can be connected to the other by a rapid application of force which caused one part to deform and engage the other.

Thus, in the prosthesis component according to the invention there are only two parts and the surgeon can easily snap in a bearing portion of appropriate thickness. Moreover, a worn bearing portion can be easily removed, it does not matter if it is damaged, and replaced by a new portion.

Preferably the main portion includes a support tray part of which is provided with inwardly projecting lugs beneath which part of said bearing portion can be located.

The bearing portion may have spaced apart lips which locate beneath said lugs and in a convenient construction the support tray has a raised rim on which said inwardly projecting lugs are provided, a lower part of said bearing portion locating on said support tray within said rim.

In order to be an appropriate shape for use in the joint the support tray and the bearing portion can be of substantially D-shape in plan view. With this arrangement the flat side of the D is located on the tibia so that it faces toward the spine of the tibia.

The lugs can be located at the spaced apart corners adjacent the ends of the curved peripheral portion on the support tray.

The bearing portion preferably has an upper part located above said lugs when in position on the support tray and the bearing portion may have a groove in its side walls beneath which the lips are formed.

0032828

The main portion of the prosthesis can be connected to the tibia in any convenient manner but preferably it is provided with a downwardly projecting spigot.

In order to provide accurate location/a second downwardly projecting and fixation location spigot can also be included.

Again, in order to assist location/the lower surface of the main and fixation portion can be provided with a patterned texture.

The upper surface of the bearing portion can be flat or dished according to the femural component with which it is to be used.

The invention also includes a total prosthesis device comprising a tibial prosthesis component as set forth above and a femural prosthesis component adapted for connection to a femur to replace a femural condyle, said femural component having a curved bearing surface for co-operation with the upper surface of the bearing portion of the tibial component.

A second tibial prosthesis component and a second femural component can also be provided to replace both tibial condyles and both femural condyles of a natural joint and in this arrangement the femural component can be interconnected if desired.

The invention can be performed in many ways but one embodiment will now be described by way of example and with reference to the accompanying drawings in which :

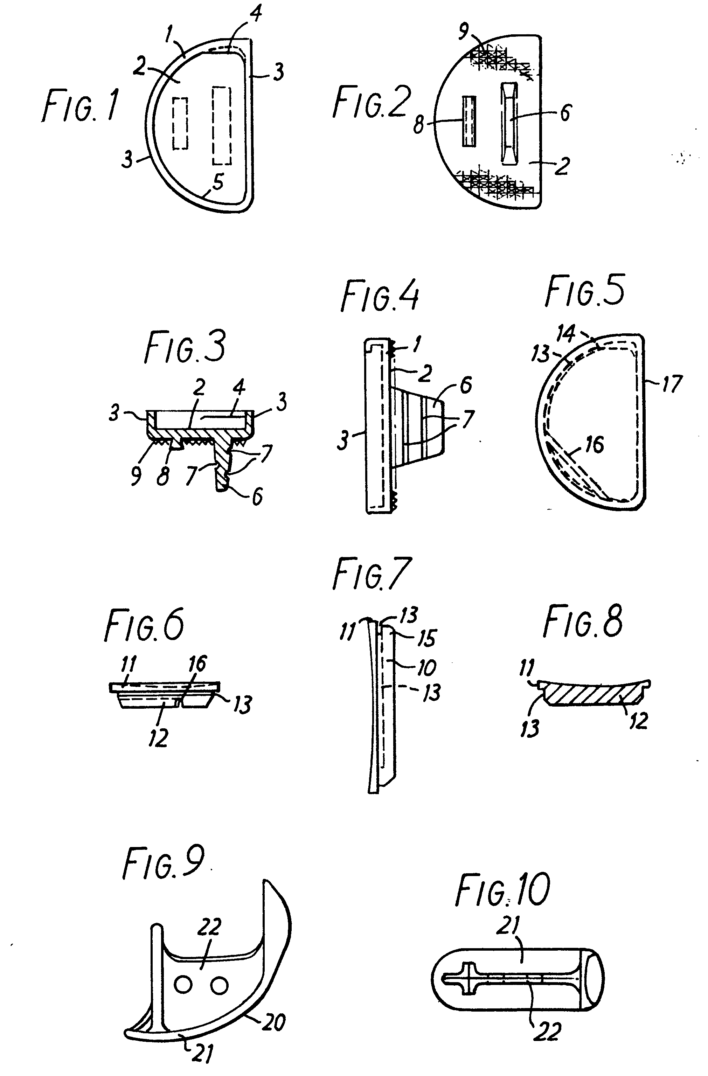

from above Figure 1 is a plan view/of a main portion of a tibial prosthesis component according to the invention.

Figure 2 is a plan view from beneath of the componenet shown in Figure 1,

Figure 3 is a cross-sectional end elevation of the componenet,

Figure 4 is a side elevation of the component shown in Figure 1,

Figure 5 is a plan view from above of a bearing portion for use with the main portion shown in Figures 1 to 4,

Figure 6 is an end elevation of the bearing portion,

Figure 7 is a side elevation of the bearing portion,

Figure 8 is a cross-sectional view through the bearing portion on the lines VIII-VIII of Figure 5,

Figure 9 is a side elevation of a femural componenet for use with the tibial component, and

Figure 10 is a plan view from above of the femural component shown in Figure 9.

As shown in the drawings the tibial prosthesis component is intended to replace a tibial condylar surface of a natural knee joint and comprises a main portion 1 as shown in Figures 1, 2, 3 and 4 and consists of a
support tray 2 which is D-shaped in plan view. This support tray 2 has a
raised rim 3 the spaced apart ends of which adjacent the end of the curved
portion of the periphery extend inwardly to provide lugs 4 and 5. The lug
5 is smaller than the lug 4 as will be apparent from Figure 1. Extending
downwardly beneath the tray portion 1 is a spigot 6 which is provided with
three transverse grooves 7 to assist fixing in position. A second smaller
donwardly projecting lug 8 is also provided this being mitre-shaped, again
to assist location. The lower surface of the tray 2 is provided with a
pyramid pattern as indicated by reference numeral 9 to assist in providing
a firm surface when a cement is also used.

This main portion is made from any suitable material such as VITALLIUM (a Co-Cr alloy) which is a Registered Trade Mark of the Applicant Company, or a stainless steel.

Arranged for connection to the main portion is a bearing portion 10 which is also D-shaped in plan view, as will be seen from Figure 5. This bearing portion comprises an upper part 11 the top surface of which is dished as will be seen from Figure 8. A lower part 12 is of smaller dimensions than the upper part 11 and a groove 13 extends around the curved periphery between the upper and lower parts and this is cut back deeper at one side where indicated by reference numeral 14. The groove provides a lip 15 extending around this curved periphery.

The lower corners of the lower part 12 are chamferred and a transverse groove 16 also extends across the lower part to assist location of the bearing part in the main portion 1.

The main portion and bearing portion are fastened together by sliding the lip 15 where the groove is undercut at 14 beneath the lug 4 on the main portion. The lower part of the bearing portion is now substantially located on the tray and within the rim 3 and the spaced apart corner is now snapped into position so that the other end of the lip fits beneath the lug 5, sufficient resilience to achieve this being provided by the transverse groove 16.

The bearing portion is now connected to the main portion with the upper part 11 of the bearing portion projecting outwardly over the rim 3.

When the prosthesis is to be used the main portion 1 is attached to the tibia with the straight side 17 of the bearing surface directed towards the spine of the tibia prior to assembly of the bearing portion. This allows selection of thickness of the bearing portion following positioning of the main portion. It will be appreciated that the condylar surface which it is to replace will have been suitably prepared in known manner.

The femural component which is for use with the tibial component described above is shown in Figures 9 and 10 and includes a curved bearing surface 20 which may have different radius, as is required and which is provided on a flange 21 connected to a suitable backing web 22, this web being shaped and provided with holes to enable it to be connected to the femur in known fashion.

If it is necessary to replace both tibial condyles then a second tibial prosthesis similar to that described above but made to the opposite hand, that is as a mirror image, can be used, once again with the straight side of the bearing surface facing towards the spine of the tibia. With this second surface a similar femural component similar to that shown in Figures 9 and 10 can again be used or if desired a combined component having two bearing surfaces 20 can be utilised.

It will be appreciated that if the femural condyl or condyles are not damaged then it is possible to use one or the pair of tibial members without the femural components.

CLAIMS:

1.      A tibial prosthesis component to replace a tibial condylar surface of a natural knee joint, said component comprising a main portion adapted for connection to the tibia and a bearing portion made from a plastics material the upper surface of which is adapted to co-operate with a femoral condylar surface of the natural joint or a prosthetic component replacing it, said bearing portion being deformable to provide a releasable snap fit connection to said main portion.

2.      A tibial prosthesis component as claimed in claim 1 in which said main portion includes a support tray part of which is provided with inwardly projecting lugs beneath which part of said bearing portion can be located.

3.      A tibial prosthesis component as claimed in claim 2 in which said bearing portion has spaced apart lips which locate beneath said lugs.

4.      A tibial prosthesis component as claimed in claim 2 or claim 3 in which said support tray has a raised rim on which said inwardly projecting lugs are provided, a lower part of said bearing portion locating on said support tray within said rim.

5.      A tibial prosthesis component as claimed in claim 4 in which said support tray and said bearing portion are substantially D-shaped in plan view.

6.      A tibial prosthesis component as claimed in claim 5 in which said lugs are located at the spaced apart corners adjacent the ends of the curved peripheral portion of the support tray.

7.      A tibial prosthesis component as claimed in any one of the preceding claims 2 to 6 in which said bearing portion has an upper part located above said lugs when in position on said support.

8.      A tibial prosthesis component as claimed in claim 7 when dependent on claims 3 to 6 in which said bearing portion has a groove in its side walls beneath which the lips are formed.

9.      A tibial prosthesis component as claimed in any one of the preceding claims in which said main portion is provided with a downwardly projecting spigot.

10.     A tibial prosthesis component as claimed in claim 9 in which said main portion is also provided with a second downwardly projecting location spigot.

11.     A tibial prosthesis component as claimed in any one of the preceding claims in which the lower surface of said main portion is provided with a patterned texture to assist location.

12.     A tibial prosthesis component as claimed in any one of the preceding claims in which the upper surface of the bearing portion is dished.

13.     A total prosthesis device comprising a tibial prosthesis component as claimed in any one of the preceding claims and a femoral prosthesis component adapted for connection to a femur to replace a femoral condyle, said femoral component having a curved bearing surface for co-operation with the upper surface of said bearing portion of the tibial component.

0032828

14.    A total prosthesis device as claimed in claim 13 in which a second tibial prosthesis component and a second femural component are provided to replace both tibial condyles and both femural condyles of the natural joint.

15.    A total prosthesis device as claimed in claim 14 in which the femural components are inter-connected.

*FIG.1*

*FIG.2*

*FIG.4*

*FIG.5*

*FIG.3*

*FIG.7*

*FIG.6*

*FIG.8*

*FIG.9*

*FIG.10*